# EUROPEAN PATENT APPLICATION

(11) **EP 0 834 289 A2**
(43) Date of publication of application: **08.04.1998**
(21) Application number: 97203010.0
(22) Date of filing: 01.10.1997
(51) Int. Cl.: A61B 17/38

(54) **Ablation catheter with an induction heated heating member**

(30) Priority: 01.10.1996 NL 1004160
(71) Applicant: Cordis Europa N.V., 9302 LJ Roden (NL)
(72) Inventor: Mulder, Rudolf Teunis, 9728 TM Groningen (NL)
(74) Representative: 't Jong, Bastiaan Jacobus

(57) **Abstract**

This invention relates to an ablation catheter comprising a tube-like basic body with a distal and a proximal end, a heating device arranged close the distal end, an energy-supply-line which is connected with the heating device and extends from the heating device, through the basic body, to the proximal end. The heating device comprises a heating member made of a magnetic material and an induction member coupled by induction with the heating member which is connected to the energy-supply-line.

## Description

The invention relates to an ablation catheter comprising a tube-like basic body with a distal and a proximal end. A heating device has been arranged close to the distal end. The catheter furthermore comprises an energy-supply-line which is connected with the heating device and extends form the heating device, through the basic body, to the proximal end.

Such an ablation catheter, whereby the heating device comprises electrodes which can be connected, via the energy-supply-line with a high-frequency electromagnetic energy-generation source, is known. The catheter is manipulated by the physician carrying out the procedure in such a way that the electrodes come into contact with the tissue which is to be ablated. By subsequently activating the source of energy, the tissue is heated and as a result ablated.

The object of the invention is to provide an ablation catheter of the type described in the preamble, which has a simple construction and can be manufactured so as to have a very small diameter.

This aim is achieved according to the invention with the ablation catheter as characterised in claim 1. By supplying a high-frequency tension via the energy-supply-line, a magnetic field alternating at a high frequency is generated in the heating member. Part of the energy inducted is dissipated in the heating member, as a result of which it is heated. The heating member can then be brought in a simply manner either directly or indirectly into contact with the tissue to be treated. As the ablating action is based on direct-heat-transfer by means of contact, very localised heating of the tissue is possible.

A very advantageous further development has been characterised in claim 2. By choosing an alloy with a Curie temperature which is equal to the required temperature of the heating member during treatment, a very simple and reliable temperature control is achieved. When the temperature of the heating member rises so as to reach the Curie temperature of the material of which this heating member has been made, the material looses its magnetic properties, so that there will be no inductive coupling with the induction member, and consequently no dissipation of energy in the material. The heating member will cool down until the Curie temperature is passed again in a downward direction, after which once again energy can be supplied via induction and the temperature will rise. Thus the temperature of the heating member will continue to rise and fall around the Curie temperature of the alloy of which it has been made.

A suitable temperature for an ablation treatment is for instance 60°C, so that preferably the measure as set out in claim 3 is employed.

As no separate temperature sensor needs to be arranged close to the heating element in the catheter in order to control
the treatment temperature, the ablation catheter according to this embodiment can be made so as to have a very small diameter, without there being any danger of overheating.

In order to achieve very sensitive action of the ablation catheter according to the invention, the measure as set out in claim 4 is preferably employed. In this case it is possible to effect a direct contact of the heating member with the tissue to be treated, as a result of which it is possible to aim the heating very carefully.

An advantageous further development has been characterised in claim 5.

The fluid under pressure conveyed through the fluid-supply-channel is heated by the heating member, so that the balloon member is filled with a heated fluid. As such a balloon, filled with a fluid, has a compliant surface, it can be pushed against the surface of the tissue to be treated, whereby over a larger surface area a good contact and consequently a good heat transfer can be achieved. Preferably, also the measure as set out in claim 2 is employed with this embodiment, so that the temperature of the fluid in the balloon member is maintained on a level with the corresponding Curie temperature.

For a good heat transfer between the fluid and the heating element the measure as set out in claim 6 is preferably employed. The central channel in the heating element can in that case connect to a central lumen in the basic body of the catheter, which can be used for supplying the fluid to the balloon member.

A suitable embodiment has furthermore been characterised in claim 7. The balloon member forms in the expanded state a sphere filled with a heated fluid at the end of the catheter. By suitable manipulation of the catheter, the balloon can be brought axially into contact with the areas of the tissue to be treated.

The invention relates to and provides as well an ablation device comprising an ablation catheter with one or more of the characterising features as set out in claims 1-7, and whereby the energy-supply-line is connected to a high-frequency AC source at the proximal end of the basic body.

The invention will be explained in greater detail in the following description with reference to the attached drawings.

Figure 1 shows partially schematically an ablation device according to the invention.

Figure 2 illustrates a partial longitudinal cross-section of an end-section of the catheter employed in the device illustrated in figure 1, as indicated by arrow II in figure 1.

Figure 3 shows a longitudinal cross-section of another embodiment corresponding to figure 2.

Figure 4 shows a longitudinal cross-section of an embodiment provided with a balloon member, corresponding to the figures 2 and 3.

Figure 5 shows a partial longitudinal cross-section of yet another embodiment.

Figure 6 shows a longitudinal cross-section of an embodiment in the form of a balloon catheter.

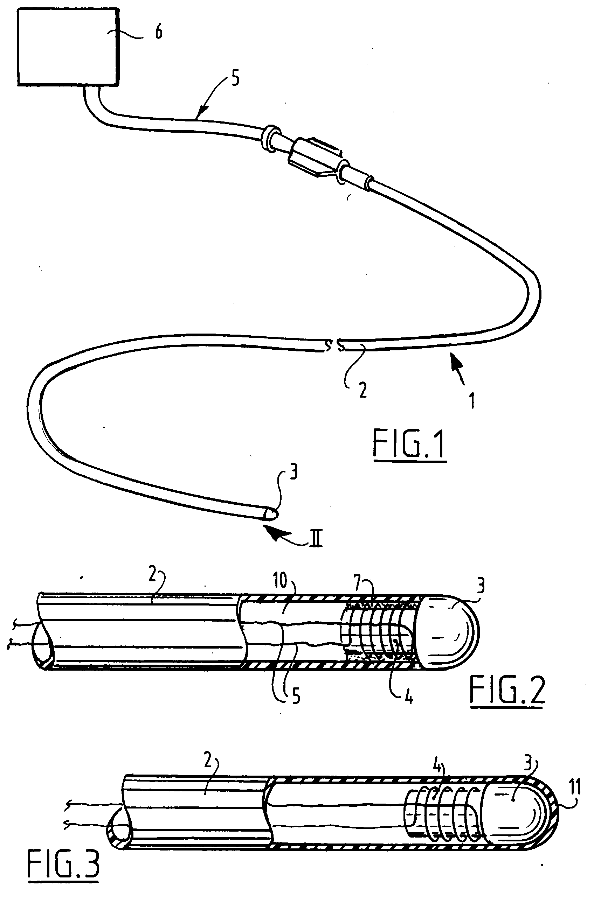

The ablation device according to the invention as illustrated in figure 1 comprises an ablation catheter 1 with a tube-like basic body 2. The tube-like basic body 2 has a distal end which, when in use, is introduced into a patient and a proximal end which remains outside the patient.

At the distal end a heating device with a heating member 3 has been arranged. The heating device has been illustrated in greater detail in figure 2.

As can be seen in figure 2, the heating device comprises the heating member 3 which has been made of a magnetic material. In general the heating member 3 has the shape of a rounded tip with the same basic diameter as the basic body 2, and comprises a shaft with a smaller diameter. Around the shaft an induction member 4 in the shape of an induction coil has been arranged, which is connected to energy-supply-lines 5. The heating member 3 with the heating coil 4 arranged around it has been fixed in the distal end of the basic body 2 by means of a filling means 7.

The energy-supply-line 5 extends from the induction member 4 at the distal end of the catheter 1 to the proximal end, where it can be connected with a high-frequency voltage source 6. When the high-frequency voltage source 6 is activated, a magnetic field alternating at a high frequency will be generated in the induction member 4, which will set the magnetic dipoles of the magnetic material of which the heating member 3 has been made into high-frequency vibrations. Due to these high-frequency vibrations of the magnetic dipoles, the temperature of the heating member 3 will increase to reach a temperature which is suitable for the intended treatment, such as ablating cardiac tissue in order to treat a tachycardia.

The heating member 3 has been made of an alloy with a Curie temperature which is suitable for the treatment, for instance a Curie temperature of 60°C. As a result the temperature of the heating member 3 will not rise above this Curie temperature. On reaching this temperature the material of which the heating member has been made will loose it magnetic properties, so that the generation of heat dependant on it stops as well. A further increase in temperature is consequently not possible.

As can be seen in figure 2, a very compact construction can be achieved as no separate temperature sensor is required. The catheter 1 according to the invention can therefore be manufactured so as to have a very small diameter.

With the embodiment of figure 2 the surface of the rounded section is not covered on the outside. As a result a very direct heat transfer and very direct local treatment is possible.

With the embodiment of figure 3 the material of which the basic body 2 has been made has been extended around the rounded tip of the heating member 3. With this embodiment the heat transfer to the tissue to be treated takes place via the end wall 11 and, as the material of which this end wall has been made is in the usual manner a plastic material, the heat transfer will take place in a more gradual fashion.

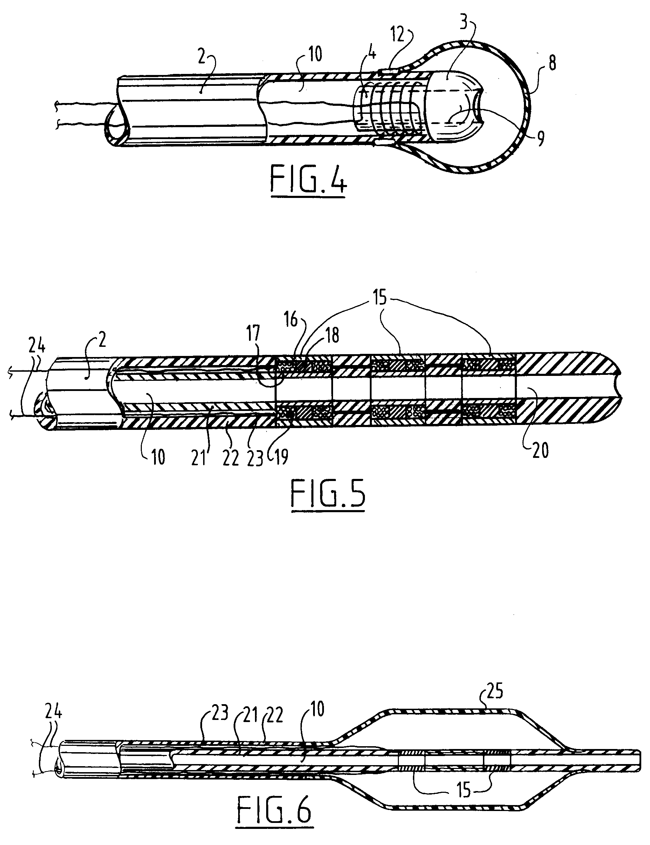

With the embodiment of figure 4 a balloon member 8 has been arranged at the distal end of the basic body 2. This balloon member comprises only one short tube-like connecting member 12, which is connected at the extreme distal end of the basic body to this basic body 2.

The heating element 3 has in this case been provided with a central channel 9, which connects to a lumen 10 in the basic body 2. Via this lumen 10 and the central channel 9 a fluid under pressure can thus be conveyed to the balloon member 8, as a result of which this balloon member can be expanded. The central channel 9 consequently forms part of the fluid-supply-channel. The fluid flowing through this central channel 9 is heated, so that the balloon 8 will be filled with a heated fluid. The fluid is further heated and its temperature is maintained through contact with the outside of the heating element 3. The balloon 8 can be positioned against the tissue to be treated and there will be heat transfer from the fluid, via the wall of the balloon member 8, to the tissue in order to ablate the latter.

With the embodiment of figure 4, the heating member 3 has also been made of an alloy with a suitable Curie temperature. Consequently the fluid inside the balloon member 8 is maintained at the temperature corresponding to the Curie temperature, as long as the induction member 4 is supplied, via the supply line, with the high-frequency electric tension.

With the embodiment of figure 5 three heating elements 15 have been received in the distal end of the basic body 2. Each heating element 15 has been made of an outer ring 16, an inner ring 17 and a number of radial connecting arms 18 in between. In the space in between the outer ring and the inner ring a coil 19 has been wound in each heating element. The coils of the successive heating elements 15 have been connected in series and the relatively proximal coil 19 has been connected to the energy-supply-lines 5. With the activated high-frequency voltage source heat will be generated in the heating elements 15, which have been made of a magnetic material, so that the annular outer surfaces of the heating elements 15 can be used for the purpose of ablating tissue.

The basic body 2 has been made up of an outer tube-like element 22 and an inner tube-like element 21 received, with some space in between, in a central lumen thereof. The connecting wires 24 which are connected to the coils in the heating elements 15 extend through the interspace 23 which has been formed in between the inner tube-like element 21 and the outer tube-like element 22.

The consequently entirely free lumen 10 connects to the inner rings 17, so that a continuous lumen from the proximal to the distal end is formed. This central lumen may for instance be used for advancing a guide wire.

In figure 6 a catheter according to the invention has been illustrated in the form of a balloon catheter. In a manner corresponding to figure 5, the basic body is made up of an inner tube-like element 21 and an outer tube-like element 22, in between which an interspace 23 has been formed. Joining up with the distal end of the outer tube-like element 22, a balloon member 25 has been arranged, which itself is connected with its distal end to the distal end of the inner tube-like element 21.

The balloon member 25 can be expanded by supplying via the interspace 23 a fluid, in particular a liquid under pressure.

As can be seen in figure 6, two heating elements 15 have been received in the inner tube-like element 21 on a level with the balloon member 25, which may for instance have been made in the same way as illustrated in figure 5. These heating elements 15 can heat the fluid in the balloon member 25, supplied via the interspace 23, after which the heat energy can be transferred to the tissue of the patient which is in contact with the balloon 25.

Also in this case the metallic parts of the heating elements 15 are preferably made of an alloy with a suitable Curie temperature.

The connecting wires 24 for the heating elements 15 have also in the case of this embodiment been led via the interspace in between the inner tube-like element 21 and the outer tube-like element 22, so that the central lumen 10 remains free for passing a guide wire through or for some other use.

The invention is not limited to the examples of embodiments illustrated in the figures. The heating elements may be embodied in many different ways. The energy-supply-line can be led, as has been shown in the figures, through a lumen but may also have been received for instance in the material of which the basic body has been made. All such variations are considered to fall within the scope of the attached claims.

## Claims

1. Ablation catheter comprising a tube-like basic body with a distal and a proximal end, a heating device arranged close to the distal end, an energy-supply-line which is connected with the heating device and extends, from the heating device, through the basic body, to the proximal end, wherein the heating device comprises a heating member made of a magnetic material and an induction member coupled by induction with the heating member which has been connected to the energy-supply-line.

2. Ablation catheter as claimed in claim 1, wherein the heating member has been made of an alloy with a Curie temperature which is higher than 37 degrees Celsius.

3. Ablation catheter as claimed in claim 2, wherein the heating member has been made of an alloy with a Curie temperature of the order of 60 degrees Celsius.

4. Ablation catheter as claimed in claim 1, wherein the heating member has at least partly an outer surface which is uncovered.

5. Ablation catheter as claimed in claim 1, furthermore comprising a balloon member, a fluid-supply-channel for the purpose of supplying a fluid under pressure to the balloon member, extending from the proximal end to the balloon member, wherein the fluid-supply-channel is bounded at least partly by the heating member.

6. Ablation catheter as claimed in claim 5, wherein the heating element comprises a central channel which forms a part of the fluid-supply-channel.

7. Ablation catheter as claimed in claim 5, wherein the balloon member comprises only one tube-like connecting member, which is connected at the extreme distal end of the basic body to the basic body.

8. Ablation device formed by an ablation catheter comprising a tube-like basic body with a distal and a proximal end, a heating device arranged close to the distal end, an energy-supply-line which is connected with the heating device and extends from the heating device, through the basic body, to the proximal end, wherein the heating device comprises a heating member made of a magnetic material, an induction member coupled by induction with the heating member which is connected to the energy-supply-line, and a high-frequency AC source connected to the energy-supply-line at the proximal end.
